# EUROPEAN PATENT SPECIFICATION

(11) **EP 3 085 385 B1**
(45) Date of publication and mention of the grant of the patent: **25.08.2021**
(21) Application number: 14871536.0
(22) Date of filing: 12.12.2014
(51) Int. Cl.: A61K 39/395, C07K 16/24, A61P 37/00

(54) **PHARMACEUTICAL COMPOSITION COMPRISING ADALIMUMAB**
PHARMAZEUTISCHE ZUSAMMENSETZUNG MIT ADALIMUMAB
COMPOSITION PHARMACEUTIQUE CONTENANT DE L'ADALIMUMAB

(30) Priority: 16.12.2013 CN 201310693338
(43) Date of publication of application: 26.10.2016
(73) Proprietor: Hisun Biopharmaceutical Co., Ltd., Zhejiang Province (CN)
(72) Inventor: FANG, Weijie, Taizhou Zhejiang 318000 (CN); WANG, Haibin, Taizhou Zhejiang 318000 (CN); QIU, Minhong, Taizhou Zhejiang 318000 (CN); ZHENG, Hongjian, Hangzhou Zhejiang 318000 (CN); YING, Yuebin, Taizhou Zhejiang 318000 (CN); BAI, Hua, Taizhou Zhejiang 318000 (CN)
(74) Representative: Kador & Partner PartG mbB
(86) International application number: PCT/CN2014/093722
(87) International publication number: WO 2015/090162

(56) References cited:
- EP-A1- 2 363 145
- WO-A1-2009/090189
- WO-A1-2010/129469
- WO-A1-2013/164837
- WO-A2-2009/073569
- WO-A2-2011/104381
- CN-A- 101 969 929

## Description

### FIELD OF THE INVENTION

The present invention belongs to the field of biotechnology, particularly, the present invention relates to a pharmaceutical composition comprising adalimumab.

### BACKGROUND OF THE INVENTION

Rheumatoid arthritis (RA) is a chronic systemic autoimmune disease characterized by arthrosynovitis. Persistent and repeated attacks of arthrosynovitis would result in the damage of cartilages and bones in joints, dysfunctions of joints, and even disability. Vasculitis lesions involve every organs in the whole body, thus the disease is also called rheumatoid disease. The onset age of about 80% patients are 20-45 years old, most of them are young adults, with the ratio of men to women being 1:2∼4. There are 0.355 billion arthritics patients around the world. In Asian region, one in six suffers from arthritis (which is the number one disease that causes disability in the world) in a certain stage of their life. At present, it is estimated that there are more than one hundred million arthritics patients in the Chinese mainland, and the number of arthritics is increasing continuously. And the number of people with rheumatoid arthritis in China reaches about 6 million.

Tumor necrosis factor α (TNF-α) is a cytokine that can kill tumor cells directly but is not toxic to normal cells. It is mainly generated from synovium macrophages and can stimulate synovium fibroblast proliferation, secrete effector molecules such as interleukin 6 (IL-6), granulocyte-macrophage colony-stimulating factor (GM-CSF), chemotactic factor, matrix metalloproteinase and prostaglandin, which plays an important role in the pathogenesis of RA. At present, TNF-α antagonist is the most effective medicine for treating RA and other similar diseases, which mainly comprises etanercept, infliximab and adalimumab and so on.

By specific binding TNF-α in a human body, adalimumab prevents TNF-α from combining with its cell surface receptor, thus blocking the biological activities of TNF-α, finally alleviating inflammatory reaction and decreasing the activation of osteoclasts, achieving the purpose of controlling and relieving symptoms and signs. The commercialization of adalimumab on the market does not only bring a new treatment strategy for rheumatoid arthritis patients, but also has an excellent clinical value and a quite great prospect in the market as adalimumab is also effective for intractable and active rheumatoid arthritis and ankylosing spondylitis that can not be treated effectively by other anti-rheumatic drugs or can not tolerate these drug treatment. Adalimumab also has the advantages of quick effect, high efficacy, good safety and tolerance. At present, the adalimumab product in the market is Humira® produced by Abbott Laboratories, US, and approved in the year 2002, and the indications approved by FDA includes: moderate-severe rheumatoid arthritis, moderate-severe juvenile rheumatoid arthritis, psoriasis, ankylosing spondylitis and moderate-severe Crohn's disease.

Adalimumab belongs to a family of fully human monoclonal antibody. "Fully human" means that it is not different from the antibody immunoglobulin IgG generated from the human body. Generally, human bodies would randomly generate various antibodies to defense various pathogens from outside. However, as the antibodies are generated randomly, antibodies treating autoantigens as the object to be attacked will also be generated. In most cases, this kind of antibodies that may attack autoantigens would be eliminated by immune system before having mature functions. Therefore, in short, antibodies to human antigens cannot be generated in a human body. Thus, the antibodies used in early treatment are usually generated in rats. Because of species difference of the source of proteins, these antibodies have short existence in human body, and the therapeutic effect is affected, and there might be a risk of allergy. Although, the proportion of antibodies from rats can be decreased by combining with human antibodies nowadays, the influence of rat-source proteins cannot be eliminated. A human antibody generated from human B cells is found by screening antibody precursors generated randomly from B cells of healthy human donors through phage expressing technology by Abbott Laboratories, US. Said human antibody, i.e. Humira®, can specifically bind to TNF-α. In other words, Humira® is a fully human TNF-α antibody found by avoiding the self-detection and eliminating mechanism of human body, which not only show an antagonistic effect to human TNF-α, but also largely decrease the immunological rejections.

At present, the adalimumab formulation, Humira® in the market is a liquid preparation comprising 50mg/mL adalimumab, 6.16mg/mL sodium chloride, 0.35mg/mL sodium citrate, 1.3mg/mL citric acid, 0.86mg/mL sodium dihydrogen phosphate dihydrate, 1.53mg/mL disodium hydrogen phosphate dihydrate, 12mg/mL mannitol, 1mg/mL polysorbate 80, pH of the solution, 5.2. Wherein adalimumab is the active ingredient, sodium chloride is the isotonic agent, citric acid and phosphate are the buffering agents, mannitol is the structural stabilizer, polysorbate 80 is the surfactant.

We found that the Humira® formulation in the market is not stable, and may degrade under long-term and accelerated experiment conditions, especially that it may generate much impurities such as aggregates and insoluble particles. While aggregates and insoluble particles are considered to be critical quality attributes (CQA) of the safety of bio-pharmaceuticals, which directly influence the medication safety of adalimumab, especially their immunogenicity, which is very important for the product of long-term medication such as adalimumab.

CN201080030083.9 discloses an adalimumab formulation that does not contain sodium chloride but contains a high concentration (>20mg/ml) of polyol such as mannitol. Compared with Humira® in the market, this new formulation has higher stability under high temperature condition. Furthermore, EP 2 363 145 A1, WO 2010/129469 A1 and WO 2011/104381 A2 disclose pharmaceutical compositions comprising adalimumab.

Like most protein molecules, adalimumab is not stable and will be subjected to various chemical and physical degradation. Compared with small molecule drugs synthesized conventionally, biomolecules have complex structures, for example high-order structures such as primary structures, secondary structures, tertiary structures. And the structures of protein, especially the high-order structures are very fragile, changes of structures happen easily, such as denaturation, aggregation and precipitation. The most fundamental requirement for the presentation of their biological activity is to maintain the high-order structure of the antibodies. These products from degradation will strongly affect the safety of biopharmaceuticals. Especially, some of the protein aggregates would stimulate immunogenic reactions in human bodies, which may decrease the therapeutic effect of the biological drug, or even cause the death of patients. For the antibody drugs, high purity products during manufacturing are required and the structure stability must also be kept during transportation, storage and usage.

Therefore, a stable adalimumab pharmaceutical composition is needed in the field of biotechnology.

### SUMMARY OF THE INVENTION

The present invention provides a pharmaceutical composition comprising adalimumab according to claim 1. Furthermore, the invention provides the pharmaceutical composition comprising adalimumab for use in treating human autoimmune diseases according to claim 3. Further embodiments are defined in the dependent claims.

Said composition can maintain the physical and chemical stability of the pharmaceutical composition for a long time, and has a viscosity and osmotic pressure that are suitable for subcutaneous administration.

Specifically, the present invention provides a pharmaceutical composition characterized in that the pharmaceutical composition comprise the following components:
(1) adalimumab 50mg/ml
(2) sucrose 83mg/ml, or trehalose 91.7mg/ml
(3) Tween 80 1mg/ml
(4) citric acid 1.3mg/ml
(5) sodium citrate 0.35mg/ml
(6) sodium dihydrogen phosphate 0.86mg/ml
(7) disodium hydrogen phosphate 1.53mg/ml
wherein the pH of the composition is 5.2 and the pharmaceutical composition does not contain sodium chloride.

It has been found surprisingly by the inventors of the present invention that the physical and chemical stability of the composition can be greatly improved compared with the prior art when sucrose or trehalose with isoosmotic pressure is chosen to be the structure stabilizer.

Furthermore, the pharmaceutical composition of the present invention does not contain sodium chloride. It has been found surprisingly by the inventors of the present invention that the stability of the adalimumab formulation that does not contain sodium chloride is significantly higher than that of the adalimumab formulation that contains sodium chloride when sucrose or trehalose with isoosmotic pressure is chosen to be the structure stabilizer.

The pharmaceutical composition of the present invention comprises 1mg/ml polysorbate 80 (Twen 80) as surfactant. The pharmaceutical composition of the present invention may further comprise surfactants comprising, but are not limited to, polysorbates, such as polysorbate 20, polysorbate 21, polysorbate 40, polysorbate 60, polysorbate 61, polysorbate 65, polysorbate 81, polysorbate 85 or mixtures thereof, poloxamer, triton, sodium dodecyl sulfate, sodium lauryl sulfate, sodium octyl glycoside, lauryl-sulphobetaine, polyethylene glycol, polypropylene glycol or mixtures thereof. The concentration of said surfactant may be 0.01-10mg/ml, preferably 0.1-2mg/ml, more preferably 1mg/ml.

The pharmaceutical composition of the present invention comprises buffer solutions comprising citric acid, sodium citrate, sodium dihydrogen phosphate and disodium hydrogen phosphate. The pharmaceutical composition of the present invention may further comprise buffer solutions comprising, but are not limited to, glycine, acetic acid/acetate, succinic acid/ succinate, ascorbic acid/ascorbate, tartaric acid/tartrate, maleic acid/ maleate, lactic acid/lactate, carbonic acid/bicarbonate, benzoic acid/benzoate, histidine, phosphoric acid or tris/tris hydrochloride, preferably the combination of citric acid/citrate and carbonic acid/bicarbonate, the pH of the composition may be adjusted to 4.0-9.0, preferably 4.5-8.0, more preferably 5.2 by the buffer solution added. The pH of the composition of the present invention is adjusted to 5.2.

The pharmaceutical composition of the present invention further comprises a pharmaceutically acceptable amount of antioxidants comprising, but are not limited to ascorbic acid, tryptophan, methionine, glutathione, sodium thiosulfate or catalase.

The pharmaceutcial composition of the present invention further comprises pharmaceutically acceptable amount of chelators comprising but are not limited to aminopolycarboxylic acid, hydroxyaminocarboxylic acid, N-substituted glycine, citric acid, nicotinamide, deferoxamine, deoxycholate and mixtures thereof, such as ethylenediamine tetraacetic acid (EDTA), diethylenetriamine pentaacetic acid (DTPA), nitrilotriacetic acid (NTA) and the salts thereof. The chelators used in the present invention can be present in the form of free acid, free base or salt of a compound, or can be present in the form of anhydrous form, hydrate or other solvate of a compound or the corresponding salt thereof.

The pharmaceutical composition of the present invention further comprises pharmaceutically acceptable amount of preservatives comprising but are not limited to metacresol, phenol, benzyl alcohol, benzalkonium chloride, phenoxyethanol or methyl parahydroxybenzoates.

The adalimumab in the pharmaceutical composition of the present invention can maintain solvable and stable within a wide range of pH value, the suitable pH value is 4.0-9.0, preferably 4.5-8.0, more preferably 5.2.

The pharmaceutical composition of the present invention can be a liquid preparation or a formulation that is prepared by lyophilized powder and water for injection through dual-chamber cartridge, and can be administered by subcutaneous injection (s.c.), intravenous injection (i.v.), intramuscular injection (i.m.) or other parenteral forms.

The pharmaceutical composition of the present invention can be used to prepare medicines to treat human autoimmune diseases, the diseases comprise rheumatoid arthritis, psoriasis, juvenile idiopathic arthritis, ankylosing spondylitis, Crohn's disease, ulcerative colitis, plaque psoriasis etc.

The features mentioned above, or the features mentioned in the examples can be randomly combined. The features disclosed in the present description can be combined with any forms of compositions, and each feature disclosed in the description can be replaced by any alternative features that provide the same, equivalent or similar purposes. Therefore, unless otherwise stated, the features disclosed are just general examples of the same or similar features.

The advantageous effects of the present invention are:
In the present invention, the stability of the pharmaceutical composition is substantially increased by using sucrose or trehalose with isoosmotic pressure as the structure stabilizer of adalimumab, and the stability is even higher during freeze-thaw test, damage of high temperature and light exposure, therefore, the present invention possesses a very broad market application potential.

### BRIEF DESCRIPTION OF THE DRAWINGS

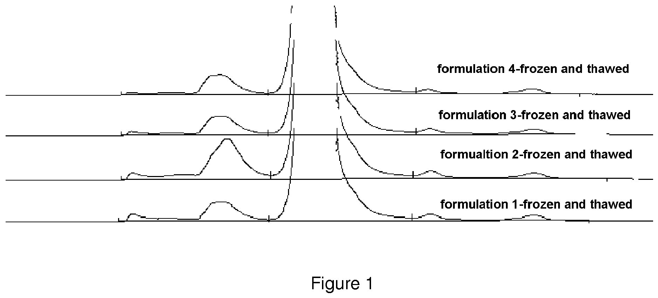

Figure 1 is the SEC-HPLC overlaid chromatography of formulations 1-4 after freeze-thaw.

### DETAILED DESCRIPTION OF THE INVENTION

Surprisingly, the inventors of the present invention have found through a large amount of experiments that in the pharmaceutical compositions comprising adalimumab, the stability of the pharmaceutical composition can be largely increased by using sucrose or trehalose with isoosmotic pressure as the structure stabilizer of the adalimumab, thus this new formulation has a very broad market application prospect.

The present invention will be further illustrated with the combination of the following examples. It should be understood that the examples are only for illustrating but not limiting the scope of the present invention. In the following examples, the experiment methods without specific conditions are generally carried out according to conventional conditions or conditions suggested by the manufacturers. Unless otherwise stated, the percentages and the parts are all based on weight.

### Example 1 Study of stability (1)

During manufacturing, transportation, storage and application, biopharmaceuticals may experience various damaging conditions such as high temperature, illumination and freeze-thawing. In order to ensure safety, they must be able to keep the structure intact, especially keep the high-order structure intact. The aggregated and insoluble particles of biotherapeutics are key factors that induce immune reaction. The appearance of biological medicines is also a very important indicator. In the pharmacopeias of countries in the world, it is provided that biological medicines must not contain visible foreign matters because the presence and the amount of particles in biological medicines are directly related to the safety of biological medicines, and they are regarded as one of the main factors that induce immune reaction of biological pharmaceuticals.

SEC-HPLC is an analytical method that carries out separation through the correlation of pore sizes of the gel pores and the sizes of the samples. In the samples, the molecules with larger molecular weight are blocked because they cannot enter gel pores and will pass through chromatographic columns along the interspaces between porous gel particles, therefore, they are firstly eluted from the column by the mobile phase. And molecules with smaller molecular weight can enter into the gel pores and receives stronger retention in the columns and can not be easily eluted, therefore, the separation of different sizes of solute molecules is achieved. The analysis to the biotherapeutics are mainly used for the measurement of the amounts of aggregates, which can be mainly represented as the loss of content of monomers and the increase of content of aggregate.

Several formulations (1-4) were designed in the present example (see table 1), wherein formulation 1 was the market product Humira® formulation; formulation 2 was a formulation that contained high concentration mannitol but did not contain sodium chloride; formulation 3 was a formulation that replaced the mannitol in formulation 1 by equivalent osmolarity of sucrose; formulation 4 was a formulation that replaced the mannitol in formulation 2 by equivalent osmolarity of sucrose.

**Table 1 The amounts of components contained in each formulation (the concentration of adalimumab was 50mg/ml)**

| Formulation | Sodium chloride(mg/ml) | sodium citrate(mg/ml) | Citric acid (mg/ml) | sodium dihydrogen phosphate(mg/ml) | disodium hydrogen phosphate(mg/ml) | Polysorbate 80 (mg/ml) | Mannitol (mg/ml) | Sucrose (mg/ml) | pH |
|---|---|---|---|---|---|---|---|---|---|
| 1 | 6.16 | 0.35 | 1.3 | 0.86 | 1.53 | 1 | 12 | / | 5.2 |
| 2 | / | 0.35 | 1.3 | 0.86 | 1.53 | 1 | 44.2 | / | 5.2 |
| 3 | 6.16 | 0.35 | 1.3 | 0.86 | 1.53 | 1 | / | 22.6 | 5.2 |
| 4 | / | 0.35 | 1.3 | 0.86 | 1.53 | 1 | / | 83 | 5.2 |

According to the concentration of every component of the formulations in table 1, 0.85ml/syringe formulations 1-4 were obtained and were studied for stability. The test results of the appearance at 0 time, the SEC-HPLC purity and the content of aggregate impurities are shown in table 2.

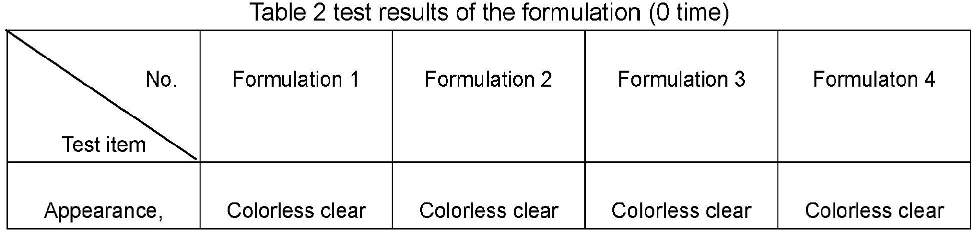

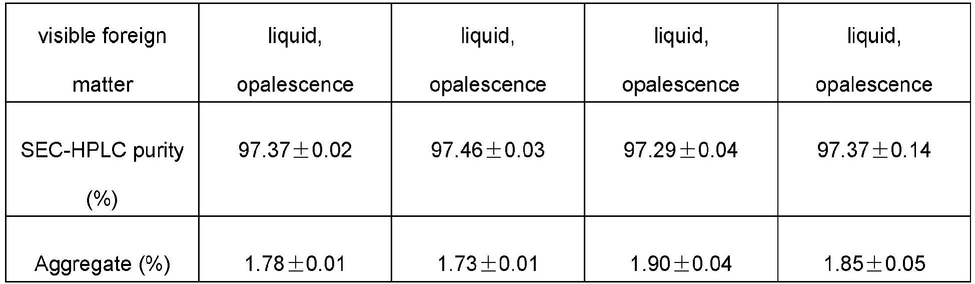

PS: The values of SEC-HPLC purity (%) and the content of aggregate (%) are all average value ± standard deviation, the same below.

### Forced degradation studies (formulations 1-4)

3 replicates of samples for each of formulations 1-4 prepared according to table 1 were subjected to high temperature, light exposure and freeze-thaw damage test, the test conditions were as follows:
High temperature test: the samples were placed in a thermostat at 40°C and were tested after 2 and 4 weeks.
Light exposure test: the samples were placed at 25°C, 600W/m² (corresponding to 110000Lx) for 4 hours and then were tested.
Freeze-thaw test: the samples were cryopreserved at -20°C for 5 hours, thawed at 4°C for 10 hours, frozen and thawed repeatedly for 8 times.

The samples of the above forced degradation studies were tested for appearance/visible foreign matters and SEC-HPLC purity (test instrument: Agilent 1200 liquid chromatograph; chromatographic column: TSK-GEL G3000 SWXL, 7.8× 300mm, TOSOH company, Japan; mobile phase: 0.2M sodium chloride, 0.02M sodium phosphate buffer solution, pH 7.4; flow rate: 0.5ml/min; column temperature: 25°C; running time: 30min; wavelength: 280nm).

### Test result for forced degradation studies

### High temperature test

3 groups of samples for each of formulations 1-4 prepared according to table 1 were placed in a thermostat at 40°C and were subjected to appearance/visible foreign matters and SEC-HPLC purity test after 2 and 4 weeks, the results are shown in table 3.

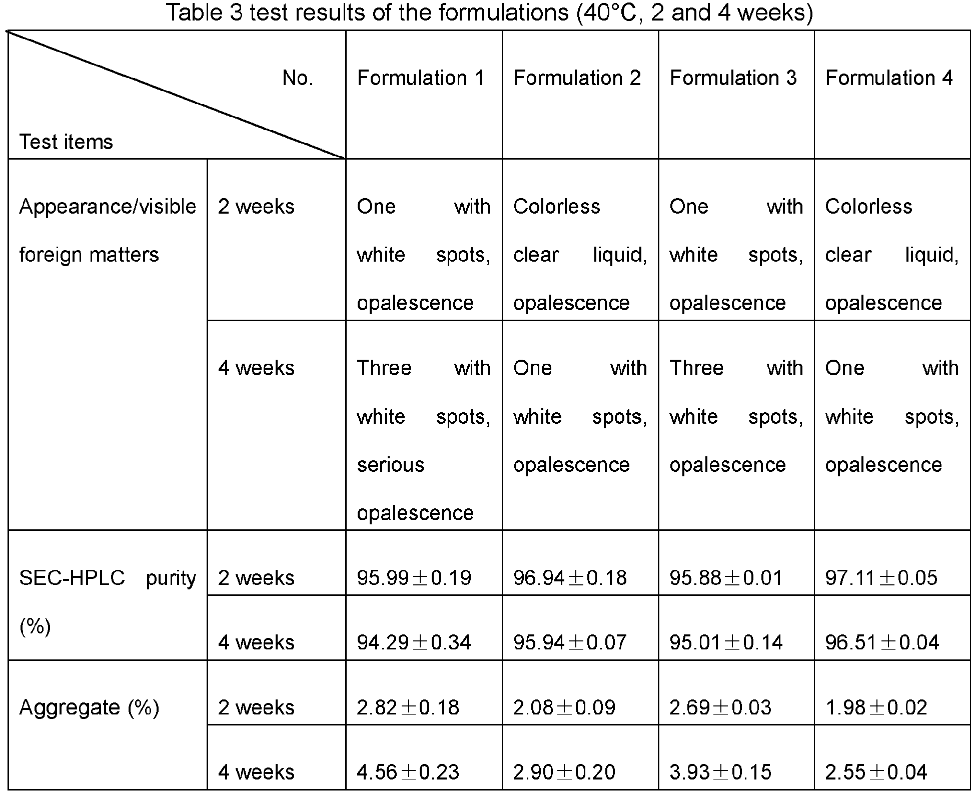

The appearance and visible foreign matters of each formulation sample after high temperature test were observed by naked eyes. 2 weeks after high temperature test, a few visible foreign matters appeared in formulation 1 and formulation 3, but not in formulation 2 and formulation 4; 4 weeks after high temperature test, a large amount of white spots appeared in formulation 1 and formulation 3, and only a few white spots appeared in one sample in both formulation 2 and formulation 4 respectively. The corresponding SEC-HPLC data also showed that the contents of aggregate after damage of formulation 3 and formulation 4 which contained sucrose as the stabilizer are lower than the corresponding formulation 1 and formulation 2 which contained mannitol as the stabilizer (table 3). The above experiment data showed that the stability of adalimumab under high temperature condition can be significantly increased by replacing sodium chloride with structure stabilizers, and the stability of the formulation that contained sucrose as the structure stabilizer (formulation 4) was significantly higher than the formulation that contained mannitol as the structure stabilizer (formulation 2).

### Light exposure test

3 groups of samples for each of formulations 1-4 prepared according to table 1 were illuminated at 25°C, 600W/m² for 4 hours, and then were tested for appearance/visible foreign matters and SEC-HPLC purity. The results are shown in table 4.

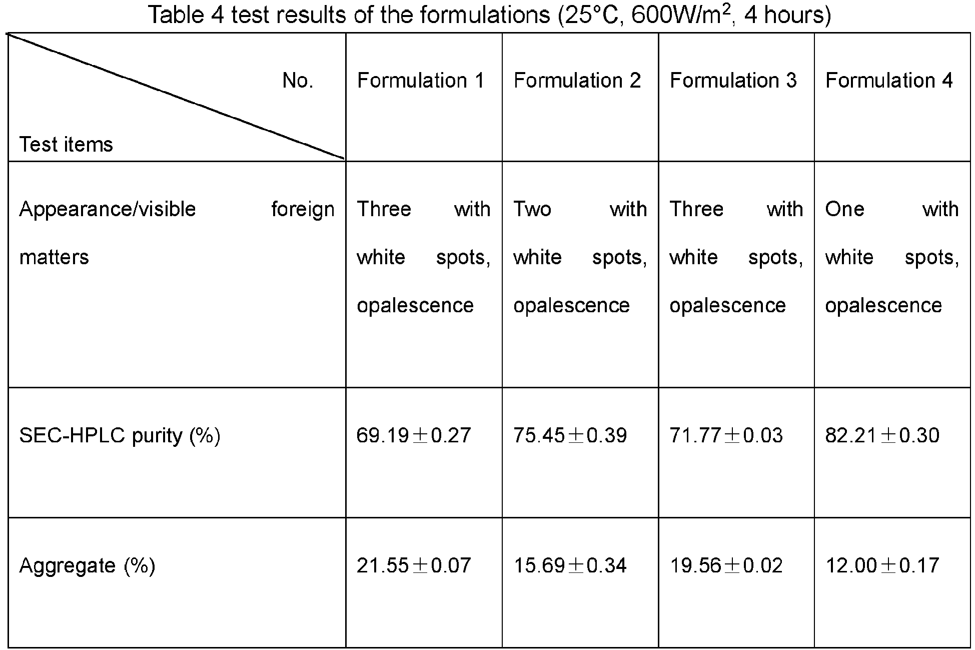

Varying degrees of white spots appeared in all formulations, but suspected white spots appeared in only one of the three samples of formulation 4 which was significantly superior to the other formulations. Suspected white spots appeared in two samples of formulation 2, formulation 1 and formulation 3 were the worst, white spots appeared in all three samples. SEC-HPLC purity trends were also identical, the marketing Humira® formulation (formulation 1) was the worst, the purity was only about 69.19%, the purity of formulation 3 was increased to about 71.77% after the mannitol of the formulation was replaced by equivalent osmolar sucrose; the SEC-HPLC purity of the formulation 2 that did not contain sodium chloride was 75.45%, and the stability of formulation 4 that did not contain sodium chloride was further strengthened after the mannitol of the formulation was replaced by equivalent osmolar sucrose (SEC-HPLC purity reached 82.21%). For the corresponding aggregate contents, formulation 1>formulation 3>formulation 2>formulation 4. The above experiment data showed that the stability of adalimumab formulation can be increased by replacing mannitol with sucrose no matter the adalimumab formulation contains sodium chloride or not.

### Freeze-thaw test

3 groups of samples for each of formulations 1-4 prepared according to table 1 were frozen at -20°C for 5 hours, and were thawed at 4°C for 10 hours and were damaged for 8 times by freezing and thawing repeatedly, then were subjected to appearance/visible foreign matters and SEC-HPLC purity test, the results are shown in table 5 and figure 1.

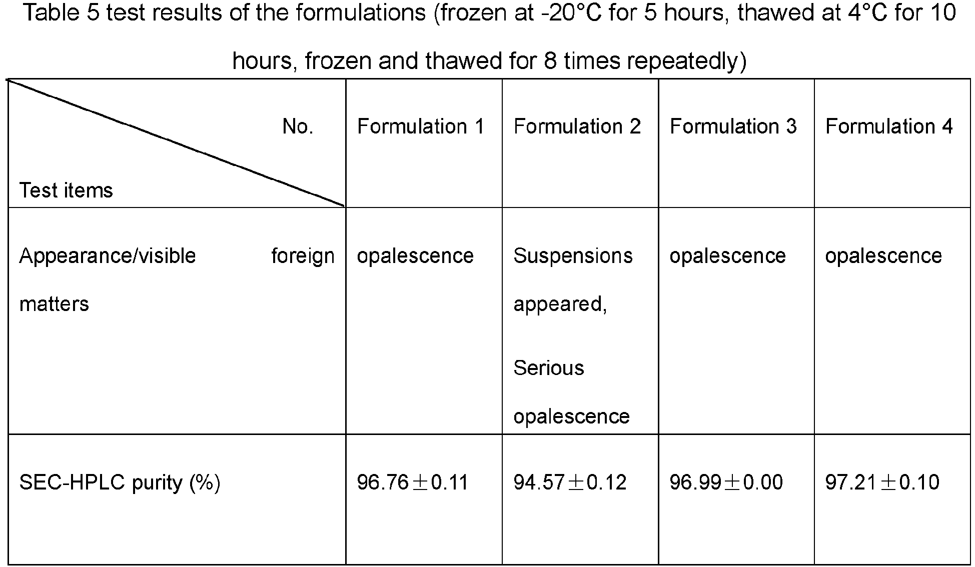

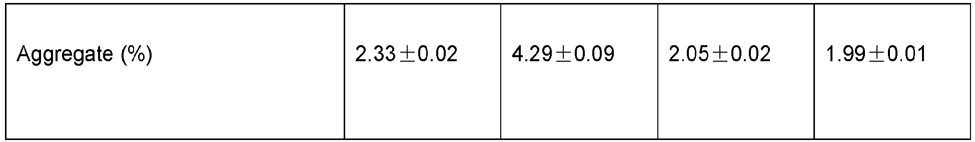

The results showed that after 8 times of damages by freezing and thawing repeatedly, the appearance/visible foreign matters and SEC-HPLC purity of formulation 4 are all superior to formulations 1 and 3, while formulation 2 significantly changed with suspension appeared and relatively serious opalescence phenomena. The SEC-HPLC purity decreased, and the increase of aggregate was very significant. This indicated that after using mannitol to replace sodium chloride in formulation 2, the adalimumab cannot be protected from being frozen and thawed and the degradation was accelerated. Formulation 4, containing isoosmolar sucrose as structure stabilizer, showed significantly superior stability compared with the formulation 2, which containing mannitol as stabilizer.

### Example 2 Study of stability (2)

Several formulations (5-11) were designed in the present example, the influences of mannitol, sucrose and trehalose on the stability under freeze-thaw of the formulations with different adalimumab concentrations were compared, in combination with the test results of formulation 2 and formulation 4 in example 1, wherein all the formulations did not contain sodium chloride, the other components in the formulations are: 0.35mg/ml sodium citrate, 1.3mg/ml citric acid, 0.86mg/ml sodium dihydrogen phosphate, 1.53mg/ml disodium hydrogen phosphate, 1mg/ml polysorbate 80, the pH of the solution was 5.2. These formulations were cryopreserved at -20°C for 5 hours, and were thawed at 4°C for 10 hours, and were frozen and thawed repeatedly for 8 times, then were subjected to SEC-HPLC purity and aggregate content test. The results are shown in table 6.

**Table 6 the influence of mannitol, sucrose and trehalose on the stability under freeze-thaw of the formulations with different adalimumab concentrations**

| Formulation | Mannitol (mg/ml) | Sucrose (mg/ml) | Trehalose (mg/ml) | The concentration of adalimumab (mg/ml) | SEC-HPLC purity (%) | aggregate (%) |
|---|---|---|---|---|---|---|
| 2 | 44.2 | / | / | 50 | 94.57 ± 0.12 | 4.29 ± 0.09 |
| 4 | / | 83 | / | 50 | 97.21 ± 0.10 | 1.99 ± 0.01 |
| 5 | / | / | 91.7 | 50 | 97.21 ± 0.10 | 1.93 ± 0.02 |
| 6 | 44.2 | / | / | 10 | 95.47 ± 0.06 | 2.27 ± 0.01 |
| 7 | / | 83 | / | 10 | 97.23 ± 0.10 | 1.85 ± 0.06 |
| 8 | / | / | 91.7 | 10 | 97.30 ± 0.14 | 1.82 ± 0.01 |
| 9 | / | / | / | 50 | 95.94 ± 0.05 | 1.94 ± 0.04 |
| 10 | 44.2 | / | / | 1 | 95.98 ± 0.28 | 4.02 ± 0.28 |
| 11 | / | 44.2 | / | 1 | 96.84 ± 0.03 | 3.16 ± 0.03 |

It can be seen from the results of table 6 that either at high (50mg/ml) or low adalimumab concentration (10mg/ml) formulation, when sucrose or trehalose with equivalent molar concentration were used as structure stabilizers, the stability of the formulation in freeze-thaw test were significantly superior to the formulation containing mannitol. Under the experiment condition when the concentration of adalimumab is 1mg/ml, the stability in freeze-thaw test of the formulation containing sucrose with the same mass concentration as mannitol was also significantly superior to the formulation containing mannitol (formulation 10 and formulation 11). Wherein, after damage by freeze-thaw, the SEC-HPLC purity of high concentration (50mg/ml) adalimumab formulation 2 that contained mannitol as the structure stabilizer was even lower than formulation 9 which did not contain any structure stabilizer.

### Example 3: Preparation of low concentration (1mg/ml) and high concentration (100mg/ml) adalimumab formulations that contain sucrose or trehalose as structure stabilizer

Formulations 12-15 were designed and formulated in the present example, which contained low concentration (1mg/ml) or high concentration (100mg/ml) adalimumab (table 7) respectively, and were used to evaluate whether sucrose and trehalose can be used to formulate low concentration or high concentration adalimumab formulations when used as a structure stabilizer. All of these formulations did not contain sodium chloride, and the other components are: 0.35mg/ml sodium citrate, 1.3mg/ml citric acid, 0.86mg/ml sodium dihydrogen phosphate, 1.53mg/ml disodium hydrogen phosphate, 1mg/ml polysorbate 80, the pH of the solution was 5.2.

**Table 7 High concentration and low concentration adalimumab formulations with sucrose or trehlaose as the structure stabilizer**

| **Formulation** | **Sucrose (mg/ml)** | **Trehalose (mg/ml)** | **The concentration of adalimumab (mg/ml)** |
|---|---|---|---|
| **12** | **83** | **I** | **1** |
| **13** | **83** | **I** | **100** |
| **14** | **I** | **91.7** | **1** |
| **15** | **I** | **91.7** | **100** |

### Example 4: Adalimumab formulations of different pH values and with sucrose or trehalose as the structure stabilizer

Formulations 16-29 were designed and formulated in the present example, which contained adalimumab formulations with different pH values (pH 4.5-8.0, table 8). These formulations do not contain sodium chloride, the other components are: 50mg/ml adalimumab, 0.35mg/ml sodium citrate, 1.3mg/ml citric acid, 0.86mg/ml sodium dihydrogen phosphate, 1.53mg/ml disodium hydrogen phosphate, 1mg/ml polysorbate 80.

**Table 8 Adalimumab formulations of different pH values and with sucrose or trehalose as the structure stabilizer**

| Formulation | Sucrose (mg/ml) | Trehalose (mg/ml) | pH |
|---|---|---|---|
| 16 | 83 | / | 4.5 |
| 17 | 83 | / | 4.8 |
| 18 | 83 | / | 5.6 |
| 19 | 83 | / | 6.0 |
| 20 | 83 | / | 6.4 |
| 21 | 83 | / | 6.8 |
| 22 | 83 | / | 8.0 |
| 23 | / | 91.7 | 4.5 |
| 24 | / | 91.7 | 4.8 |
| 25 | / | 91.7 | 5.6 |
| 26 | / | 91.7 | 6.0 |
| 27 | / | 91.7 | 6.4 |
| 28 | / | 91.7 | 6.8 |
| 29 | / | 91.7 | 8.0 |

### Example 5: Preparation of adalimumab formulations with different sucrose or trehalose concentrations

Adalimumab formulations 30-35 with different concentrations of sucrose or trehalose as the structure stabilizer were designed and formulated in the present example (table 9). Wherein these formulations do not contain sodium chloride, the other components in the formulations are: 50mg/ml adalimumab, 0.35mg/ml sodium citrate, 1.3mg/ml citric acid, 0.86mg/ml sodium dihydrogen phosphate, 1.53mg/ml disodium hydrogen phosphate, 1mg/ml polysorbate 80.

**Table 9 Adalimumab formulations with different concentrations of sucrose or trehalose as the structure stabilizer**

| Formulations | Sucrose (mg/ml) | Trehalose (mg/ml) |
|---|---|---|
| 30 | 22.6 | / |
| 31 | 50 | / |
| 32 | 200 | / |
| 33 | / | 25 |
| 34 | / | 50 |
| 35 | / | 200 |

### Example 6: Preparation of adalimumab formulations with different contents of polysorbate 80 and with sucrose or trehalose as the structure stabilizer

Formulations 36-45 were designed and formulated in the present example, which contained adalimumab formulations with different concentrations of polysorbate 80 (table 10). Wherein these formulations do not contain sodium chloride, the other components in the formulations are: 50mg/ml adalimumab, 0.35mg/ml sodium citrate, 1.3mg/ml citric acid, 0.86mg/ml sodium dihydrogen phosphate, 1.53mg/ml disodium hydrogen phosphate, pH 5.2.

**Table 10 Adalimumab formulations that contained sucrose or trehalose and with different concentrations of polysorbate 80**

| Formulations | Sucrose (mg/ml) | Trehalose (mg/ml) | Polysorbate 80 (mg/ml) |
|---|---|---|---|
| 36 | 83 | / | 0.01 |
| 37 | 83 | / | 0.1 |
| 38 | 83 | / | 0.5 |
| 39 | 83 | / | 2 |
| 40 | 83 | / | 5 |
| 41 | / | 91.7 | 0.01 |
| 42 | / | 91.7 | 0.1 |
| 43 | / | 91.7 | 0.5 |
| 44 | / | 91.7 | 2 |
| 45 | / | 91.7 | 5 |

## Claims

1. A pharmaceutical composition, **characterized in that** the pharmaceutical composition comprises the following components:
(1) adalimumab 50mg/ml
(2) sucrose 83mg/ml, or trehalose 91.7mg/ml
(3) Tween 80 1mg/ml
(4) citric acid 1.3mg/ml
(5) sodium citrate 0.35mg/ml
(6) sodium dihydrogen phosphate 0.86mg/ml
(7) disodium hydrogen phosphate 1.53mg/ml
wherein the pH of the composition is 5.2 and the pharmaceutical composition does not contain sodium chloride.

2. The pharmaceutical composition according to claim 1, **characterized in that** the pharmaceutical composition is a liquid preparation or a formulation that is prepared by lyophilized powder and water for injection through dual-chamber cartridge.

3. The pharmaceutical composition according to claim 1 or 2 for use in treating human autoimmune diseases.

4. The pharmaceutical composition for use according to claim 3, **characterized in that** the human autoimmune diseases comprise rheumatoid arthritis, psoriasis, juvenile idiopathic arthritis, ankylosing spondylitis, Crohn's disease, ulcerative colitis and plaque psoriasis.

5. The pharmaceutical composition for use according to claim 3 or 4, wherein the composition can be administered by subcutaneous injection (s.c.), intravenous injection (i.v.), intramuscular injection (i.m.) or other parenteral forms.

## Patentansprüche

1. Pharmazeutische Zusammensetzung, **dadurch gekennzeichnet, dass** die pharmazeutische Zusammensetzung die folgenden Komponenten umfasst:
(1) Adalimumab 50 mg/ml;
(2) Saccharose 83 mg/ml oder Trehalose 91,7 mg/ml;
(3) Tween 80 1 mg/ml;
(4) Zitronensäure 1,3 mg/ml;
(5) Natriumcitrat 0,35 mg/ml;
(6) Natriumdihydrogenphosphat 0,86 mg/ml;
(7) Dinatriumhydrogenphosphat 1,53 mg/ml;
wobei der pH-Wert der Zusammensetzung 5,2 beträgt und die pharmazeutische Zusammensetzung kein Natriumchlorid enthält.

2. Pharmazeutische Zusammensetzung nach Anspruch 1, **dadurch gekennzeichnet, dass** die pharmazeutische Zusammensetzung ein flüssiges Präparat oder eine Formulierung ist, die durch lyophilisiertes Pulver und Wasser zur Injektion durch eine Zweikammerkartusche hergestellt wird.

3. Pharmazeutische Zusammensetzung nach Anspruch 1 oder 2 zur Verwendung bei der Behandlung von menschlichen Autoimmunkrankheiten.

4. Pharmazeutische Zusammensetzung zur Verwendung nach Anspruch 3, **dadurch gekennzeichnet, dass** die menschlichen Autoimmunerkrankungen rheumatoide Arthritis, Psoriasis, juvenile idiopathische Arthritis, Spondylitis ankylosans, Morbus Crohn, Colitis ulcerosa und Plaque-Psoriasis umfassen.

5. Pharmazeutische Zusammensetzung zur Verwendung nach Anspruch 3 oder 4, wobei die Zusammensetzung durch subkutane Injektion (s.c.), intravenöse Injektion (i.v.), intramuskuläre Injektion (i.m.) oder andere parenterale Formen verabreicht werden kann.

## Revendications

1. Composition pharmaceutique, **caractérisée en ce que** la composition pharmaceutique comprend les composants suivants :
(1) adalimumab 50 mg/ml
(2) saccharose 83 mg/ml, ou tréhalose 91,7 mg/ml
(3) Tween 80 1 mg/ml
(4) acide citrique 1,3 mg/ml
(5) citrate de sodium 0,35 mg/ml
(6) dihydrogénophosphate de sodium 0,86 mg/ml
(7) monohydrogénophosphate de sodium 1,53 mg/ml
le pH de la composition étant de 5,2 et la composition pharmaceutique ne contenant pas de chlorure de sodium.

2. Composition pharmaceutique selon la revendication 1, **caractérisée en ce que** la composition pharmaceutique est une préparation liquide ou une formulation qui est préparée par de la poudre lyophilisée et de l'eau pour injection par le biais d'une cartouche à chambre double.

3. Composition pharmaceutique selon la revendication 1 ou 2 pour utilisation dans le traitement de maladies auto-immunes humaines.

4. Composition pharmaceutique pour utilisation selon la revendication 3, **caractérisée en ce que** les maladies auto-immunes humaines comprenant la polyarthrite rhumatoïde, le psoriasis, l'arthrite juvénile idiopathique, la spondylite ankylosante, la maladie de Crohn, la colite ulcéreuse et le psoriasis en plaques.

5. Composition pharmaceutique pour utilisation selon la revendication 3 ou 4, la composition pouvant être administrée par injection sous-cutanée (s.c.), injection intraveineuse (i.v.), injection intramusculaire (i.m.) ou d'autres formes parentérales.
